# EUROPEAN PATENT SPECIFICATION

(11) **EP 3 729 094 B1**
(45) Date of publication and mention of the grant of the patent: **07.12.2022**
(21) Application number: 18836615.7
(22) Date of filing: 21.12.2018
(51) Int. Cl.: G01N 33/569, G01N 33/68

(54) **METHODS AND MATERIALS FOR THE DETECTION OF LATENT TUBERCULOSIS INFECTION**
METHODEN UND ZUSAMMENSETZUNGEN ZUR NACHWEIS VON DER LATENTEN INFEKTIONSPHASE EINES TUBERKULOSE INFEKTIONS
PROCEDES ET COMPOSITIONS DE DETECTION DE LA PHASE LATENTE D'INFECTION DE LA TUBERCULOSE

(30) Priority: 22.12.2017 GB 201721761
(43) Date of publication of application: 28.10.2020
(73) Proprietor: MJO Innovation Ltd, London WC2A 1LS (GB)
(72) Inventor: LALVANI, Ajit, London W2 1PZ (GB); HALLIDAY, Alice, Bristol BS8 1TD (GB)
(74) Representative: Potter Clarkson
(86) International application number: PCT/GB2018/053757
(87) International publication number: WO 2019/122929

(56) References cited:
- WO-A2-2015/171552
- TOIDI ADEKAMBI ET AL: "Biomarkers on patient T cells diagnose active tuberculosis and monitor treatment response", JOURNAL OF CLINICAL INVESTIGATION, vol. 125, no. 5, 30 March 2015 (2015-03-30), pages 1827-1838, XP055555835, GB ISSN: 0021-9738, DOI: 10.1172/JCI77990
- Katalin Wilkinson: "CORRESPONDENCE Activation Profile of Mycobacterium tuberculosis-Specific CD4 1 T Cells Reflects Disease Activity Irrespective of HIV Status", Am J Respiratory & Critical Care Medicine, 1 June 2016 (2016-06-01), pages 1307-1310, XP055555465, Retrieved from the Internet: URL:https://www.ncbi.nlm.nih.gov/pmc/artic les/PMC4910903/pdf/rccm.201601-0116le.pdf [retrieved on 2019-02-12]
- CATHERINE RIOU ET AL: "A Subset of Circulating Blood Mycobacteria-Specific CD4 T Cells Can Predict the Time to Mycobacterium tuberculosis Sputum Culture Conversion", PLOS ONE, vol. 9, no. 7, 21 July 2014 (2014-07-21), page e102178, XP055555452, DOI: 10.1371/journal.pone.0102178

## Description

The present invention relates to methods for determining latent tuberculosis (TB) infection (LTBI) status.

Worldwide there are approximately 1.3 million deaths due to active tuberculosis (TB) every year (World Health Organisation (WHO) 2017) and in the developed world, including the UK, rates of TB are still unacceptably high, especially in certain high incidence areas, such as in large urban areas (e.g. London) (Tuberculosis in England 2017; Public Health England),

Identification of LTBl and prevention of subsequent progression to active TB disease is the cornerstone of TB control in most high-income, low-incidence countries (such as in Europe and the USA). However, preventative treatment of individuals identified as having LTBI is long (≥3 months) and can have significant side effects. It is therefore a clinical imperative to identify those persons at greatest risk of progression from LTBI to active disease.

The existing standard of care for identifying individuals at risk of developing ATB is to use either the tuberculin skin test (TST), or an interferon gamma release assay (IGRA), which can be used to diagnose LTBI. Those with a positive test are offered preventative therapy to prevent active TB. Using either of these tests, the number needed to treat to prevent one case of active TB is >20.

There is currently no test which is able to investigate LTBI status and identify individuals with LTBI who are at greater risk of progressing to active disease, If such a test was available, it would give clinicians and nurses more confidence about how to recommend treatment for individuals with latent TB, and would greater reduce the number of people needed to treat to prevent one case of active TB disease.

Unlike, for example HIV infection, where the amount of virus in the body (plasma or other compartments) is directly quantifiable using PCR, there is no comparable test to directly measure mycobacterial (e.g. *Mycobacterium* tuberculosis) pathogen load. A widely used TB diagnostic assay, the mantoux or tuberculin skin test (TST), uses an immune-based approach to demonstrate the presence of infection with *Mycobacterium tuberculosis* (MTB). The TST has poor specificity especially in those who are BCG-vaccinated (Diel, Goletti *et al,* 2011) and poor sensitivity especially in those who are immunocompromised e.g. with HIV infection (Cobeiens. Egwaga *et al.* 2006).

Investigation of both active and latent TB infection may involve multiple invasive, time consuming and expensive tests such as CT with contrast, bronchoscopy, biopsy or PET scanning. A blood test that could reduce the need for such intensive investigation would therefore be of great benefit to both patients and healthcare providers,

Treatment of active TB requires a longer, more complex and more toxic treatment regime than the chemoprophyiaxis used for LTBI, however both can be associated with potentially life-threatening toxicity. Many therapeutics currently used to treat mycobacterial infection have high associated morbidity e.g. peripheral neuropathy and mortality e.g. liver necrosis. Therefore, use of the appropriate shortest and most effective therapeutic regime is vital.

As most patients with LTBI will never progress to active TB, there is an opportunity to improve care by identifying those LTBI patients most likely to progress and prioritising them to receive treatment Those unlikely to progress would thus be able to avoid taking chemoprophyiaxis altogether.

Consequently, there is a continued need for diagnostic tests able to determine latent TB infection status and particularly to distinguish these two groups. Given that the likelihood of progression is greatest in the first 2-3 years following MTB infection, correlates of time since exposure can also provide a means for risk stratification. The inventors herein describe an immune based test which is surprisingly able to determine latent TB infection status and particularly identify individuals with LTBI who have a greater risk of development to active TB and/or determine the amount of time since an individual was infected with TB. Thus, the test can be used to risk stratify patients with LTBI for preventative treatment for active TS.

In a first aspect of the invention there is provided an in vitro method of determining the latent tuberculosis (TB) infection status in an individual comprising:
(i) providing a sample comprising T~cells;
(ii) exposing the sample of (i) to one or more TB antigens;
(iii) identifying T-cells in the sample that are CD4 positive and secrete IFN-γ in response to TB antigens;
(iv) identifying those cells of (iii) which are also HLA-DR positive; and
(v) calculating the cells identified in (iv) as a percentage of those identified in (iii);
wherein the identification of cells in (iv) and/or the percentage of cells calculated in (v) correlates to latent TB infection status of the individual, and wherein steps (iii) and (iv) can be carried out either sequentially or simultaneously, wherein the latent TB infection status determined by the method corresponds to the risk of the latent TB infection progressing to an active TB infection, and wherein the risk of the latent TB infection progressing to an active TB infection is proportional to the percentage value calculated in step (v) of the method.

Steps (i) and (ii) may be performed as a distinct methodology e.g. as part of an alternative assay such as an IGRA (interferon-gamma release assay), ELiSpot (Enzyme-Linked Immunosorbent Spot), or T-Spot,TB assay and consequently may be performed at a different time or using a different piece of equipment (e.g. well plate) or even a different physical location to the remaining steps.

in an additional or alternative embodiment the cells identified in step (iii) of the methods are additionally CD3 positive.

In an additional or alternative embodiment the cells identified in step (iii) of the methods are additionally CD8 negative.

"TB" is used interchangeably herein with "tuberculosis", It is intended to include TB caused by any Mtb organisms including *Mycobacterium tuberculosis, Mycobacterium* bovis and *Mycobacterium africanum.* In one particular embodiment the TB is caused by *Mycobacterium tuberculosis.*

By "individual" we refer to any organism capable of being infected with TB. Preferably the individual is a mammal, more preferably selected from cattle, badgers and humans. Most preferably the individual is a human.

By "TB antigens" we refer to any Mtb protein or peptide preparation capable of eliciting an immune response. Examples of Mtb antigens are tuberculosis purified protein derivative (PPD), ESAT-6, CFP-10, EspC, TB7.7, Rv3879c, Rv3615c, Rv3873, Rv3878, PE/PPE proteins, Ag85A/B and TB10.4, Heparin-Binding Haemagglutinin, Rv0440, Rv3875/Rv3874, Rv0140, Rv0384c, Rv2662, Rv3223c, Rv3307, Rv3862c, Rv0079, Rv0081, Rv0574c, Rv1733c, Rv1734c, Rv1998, Rv2006, Rv2007c, Rv2028c, Rv2627c, Rv2629, Rv2630, Rv3132c, Rv0867c, Rv1009, Rv1884c, Rv2389c. Any other TB antigen known in the art could also be used.

In one particular embodiment, the Mtb antigens comprise RD-1 antigens.

in an additional or alternative embodiment the Mtb antigens comprise one or more of ESAT-6, CFP-10. Rv3615c, and Rv3879c.

In an additional or alternative embodiment, a positive and/or negative control is included as part of the method. For a positive control, positive stimulation (with, for example, PMA and/or ionomycin (e.g. 1 ug/ml and 100 ug/ml respectively)) may be used in step (ii). For a negative control, there may be no stimulation in step (ii), i.e. no TB antigens are added at step (ii).

In an additional or alternative embodiment the cells in step (iii) are additionally identified as live T-cells. They may be identified as live cells by any method known in the art, such as the use of either a dead cell marker or a live cell marker. Such markers are well known in the art (e.g. UVE/DEAD^{®} fixable dead cell stain kits).

Dead cell markers label internal cellular structures via disrupted membranes in dead cells. Other dyes that could be used as dead cell markers are impermeant nucleic acid dyes e,g. EMA, DAPI, SYTOX^{®}Blue/Green/Red, Ethidium homodimer-1, Propidium iodide, 7-AAD, monomeric cyanine dyes, Annexin V dyes. Not all of these dyes are suitable for use with formaldehyde fixation unlike the LIVE/DEAD^{®} fixable dead cell stain kits.

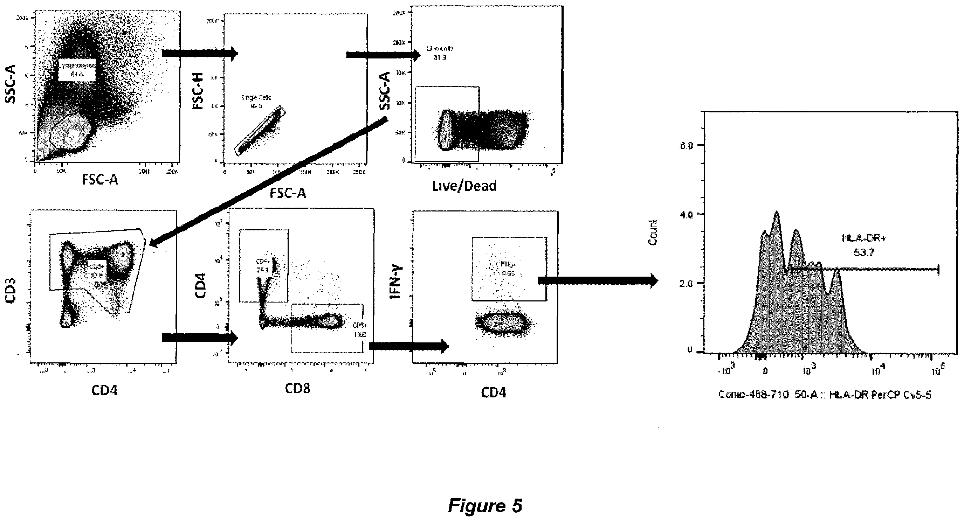

The particular subsets of T-cells as specified in steps (iii) and (iv) can be identified by any suitable technique known in the art. However, a preferred technique is that of multi-parameter flow cytometry. This technique is well known in the art as a method which can be used to determine the amount of cells of a certain type in a sample by criteria such as cell phenotype and/or cell function and using a cocktail of visually tagged antibodies specific for multiple markers of cell phenotype or function. An example of a gating strategy which may be used is shown in Figure 5. Firstly, lymphocytes are identified using forward and side scatter, then singlets are selected, followed by live cells, CD3+ calls (T cells), then CD4+ cells. The lFNγ+ cells are selected, and the proportion of HLA-DR positive cells is determined using a histogram plot. All gates are set using FMO controls.

In an additional or alternative embodiment, steps (iii) and/or (iv) of the methods of the first aspect of the invention are performed by multi-parameter flow cytometry.

A preferred method of multi-parameter flow cytometry will involve reduction of the number of fluorophore detectors required.

In an additional or alternative embodiment the risk of a latent TB infection progressing to an active TB infection is determined by the Identification of one or more cells in step (iv). In other words, if one or more cells are identified in step (iv), the individual is at risk of progressing from a latent TB infection to active TB.

In an additional or alternative embodiment the risk of a latent TB infection progressing to an active TB infection is proportional (positively correlated) to the percentage value calculated in step (v) of the method. For example, a higher percentage value calculated in step (v) correlates with an increased risk of a latent TB infection progressing to an active TB infection.

In an additional or alternative embodiment there is a high risk of the latent TB infection progressing to an active TB infection when the percentage calculated in step (v) is greater than a cut off value. In other words, if the percentage calculated in step (v) is greater than a particular percentage value, the individual is determined as being at high risk of progressing from a latent to an active TB infection.

By "high risk", we include the meaning that the individual is at a sufficient level of risk of progressing to an active TB infection to warrant receiving appropriate preventative treatment. For example, the method of the invention could be used stratify "high risk" individuals for preventative treatment for active TB.

The cut off value may be determined by a person skilled in this field depending on various criteria, such as the necessary sensitivity and selectivity levels required for that particular patient or group of patients.

In order to derive a cut-off for this assay, a skilled user would first test the performance of the assay in their target population of interest. Given the variability in relative risk of progression to active disease in different populations, as well as biological variation in HLA-DR expression, and laboratory practices, the optimal cut-off for identifying those at higher risk of progression will vary depending on the setting. In addition, users will want to define their cut off to prioritise either high sensitivity, or high specificity, which will depend on different factors, such as the particular patient or patient group, e.g. age, HIV status, and/or whether or not they are taking immunosuppressants. For example, two particular cut off values which could be used based on the data in Example 1 are 28% and 32.5%, depending on the preferred sensitivity and selectivity of the user.

For example, if the cut off value selected is 28%, an individual would be determined to be at high risk of progressing to active TB and/or stratified as requiring preventative treatment for active TB if the percentage value calculated in step (v) of the methods of the invention is greater than 28%.

in an additional or alternative embodiment, the individual is determined as being at high risk of progressing from a latent to an active TB infection when the percentage calculated in step (v) is greater than a cut off value of between 10% and 50%, i.e. the cut off percentage value used may be any value between 10 and 50%. In other words, the cut off value may be selected as a particular percentage value within that range, e.g. 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50% could be used as a cut off value. However, it may not be a whole number and the cut off value could be calculated to one or more decimal places.

In an additional or alternative embodiment, the individual is determined as being at high risk of progressing from a latent to an active TB infection when the percentage calculated in step (v) is greater than a cut off value selected from between 20% and 50%.

In an additional or alternative embodiment, the individual is determined as being at high risk of progressing from a latent to an active TB infection when the percentage calculated in step (v) is greater than a cut off value selected from between 25% and 50%,

In an additional or alternative embodiment, the individual is determined as being at high risk of progressing from a latent to an active TB infection when the percentage calculated in step (v) is greater than a cut off value selected from between 10% and 40%.

In an additional or alternative embodiment, the individual is determined as being at high risk of progressing from a latent to an active TB infection when the percentage calculated in step (v) is greater than a cut off value selected from between 10% and 35%.

In an additional or alternative embodiment, the individual is determined as being at high risk of progressing from a latent to an active TB infection when the percentage calculated in step (v) is greater than a cut off value selected from between 10% and 30%.

In an additional or alternative embodiment, the individual is determined as being at high risk of progressing from a latent to an active TB infection when the percentage calculated in step (v) is greater than a cut off value selected from between 20% and 40%.

In an additional or alternative embodiment, the individual is determined as being at high risk of progressing from a latent to an active TB infection when the percentage calculated in step (v) is greater than a cut off value selected from between 25% and 40%.

In an additional or alternative embodiment, the individual is determined as being at high risk of progressing from a latent to an active TB infection when the percentage calculated in step (v) is greater than a cut off value selected from between 25% and 35%.

In an additional or alternative embodiment, the individual is determined as being at high risk of progressing from a latent to an active TB infection when the percentage calculated in step (v) is greater than a cut off value selected from between 20% and 35%.

In an additional or alternative embodiment, the individual is determined as being at high risk of progressing from a latent to an active TB infection when the percentage calculated in step (v) is greater than a cut off value selected from between 25% and 30%.

In an additional or alternative embodiment, the individual is determined as being at high risk of progressing from a latent to an active TB infection when the percentage calculated in step (v) is greater than a cut off value of 10, 11, 12, 13, 14, 15, 16, 17, 18, 19, 20, 21, 22, 23, 24, 25, 26, 27, 28, 29, 30, 31, 32, 33, 34, 35, 36. 37, 38, 39, 40, 41, 42, 43, 44, 45, 46, 47, 48, 49 or 50%, or a cut off value failing within any particular range of these values. For example, the lower end of the range of values which could be used as the cut off may be selected from 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50%, and the higher end of the range of values which could be used as the cut off may be selected from 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50%, ensuring that the higher end of the range is a higher number than the lower end of the range. In practice, the actual cut off value used may not be a whole number and could be calculated to one or more decimal places.

In an additional or alternative embodiment of the invention the latent TB infection status identified by the method of the first aspect of the invention corresponds to the amount of time elapsed since the individual was originally infected with TB. In other words, the method of the invention is able to determine the relative time since the individual was originally infected with TB.

In an additional or alternative embodiment the time elapsed since the individual was originally infected with TB is inversely proportional (negatively correlated) to the percentage value calculated in step (v). For example, a higher percentage value calculated in step (v) correlates to a shorter amount of time since the individual was originally infected with TB.

A more recently acquired asymptomatic infection is more likely to progress to active TB disease than an infection acquired longer ago. Therefore, the proportion or frequency of CD4+IFNγ+HLA-DR+ T-ceils also correlates with the recentness of TB exposure.

The methods of the invention allow determination of the TB status of an individual regardless of whether or not the individual is also infected with HIV. In other words, the methods of the invention function independently of the HIV status of the individual

In an additional or alternative embodiment the individual is infected with HIV. In other embodiments the individual is not infected with HIV, or the HIV infection status of the individual is unknown.

The methods of the invention allow determination of the TB status of an individual regardless of the site of the TB infection. In other words, the methods of the invention function independently of the site of the TB infection.

For example, the individual may be or have been infected with TB in the lung (pulmonary) or at any other site (extrapulmonary), or the site of infection may be unknown.

Examples of extrapulmonary infection sites include the pleura (e.g. in tuberculous pleurisy), the central nervous system (e.g. in tuberculous meningitis), the lymphatic system (e.g. in scrofula of the neck), the genitourinary system (e.g. in urogenital tuberculosis), the bones and joints (e.g. in Pott's disease of the spine), the skin, and gastrointestinal manifestations, among others.

In an additional or alternative embodiment the individual has been infected with TB at any site. In other words, the site of infection may be pulmonary and/or extrapulmonary. In certain embodiments the site of infection is unknown.

in an additional or alternative embodiment of the invention the sample provided in step (i) of the methods of the first aspect of the invention is a blood sample, preferably a peripheral blood mononuclear cell (PBMC) sample. The sample may comprise whole blood, or a fraction separated from blood. Alternatively, a bronochoalveolar lavage (BAL) or cerebral spinal fluid (CSF) sample may be used.

In an additional or alternative embodiment the sample may be frozen and thawed before use.

In certain embodiments of the invention, the method is 60-100% sensitive and/or 60-100% specific. In one embodiment the method is 89% sensitive and/or 68% specific. In another embodiment the method is 67% sensitive and/or 84% specific. Sensitivity and specificity are calculated using receiver operator curve analysis. This calculates a range of sensitivity and specificities depending on which percentage cut-off is drawn, see Example 1.

In an additional or alternative embodiment of the invention the T-celis identified in step (iii) are identified as live T-cells, preferably by use of a dead cell marker.

In an additional or alternative embodiment of the invention an additional step (ii-a) is performed between steps (ii) and (iii) to block the release of cytokines, preferably by adding a golgi-inhibitor, e.g. Brefeidin A and/or Monensin.

In an additional or alternative embodiment of the invention an additional step is performed in order to determine that the sample is obtained from an individual infected with latent TB. In other words, an additional step is performed to determine that the individual is infected with latent TB. The additional step may be performed before step (i). The additional step may be performed using an ELISpot platform, an interferon gamma release assay (IGRA) and/or a tuberculin skin test. Alternatively, the additional step may be performed using any method based on TB-specific antigens to determine the presence of latent TB infection, or any other assay that is able to detect Mtb infection.

In an additional or alternative embodiment of the invention there is an absence of clinical and/or radiological features of active TB in the individual. With clinical features mainly being fever, night sweats, weight loss, coughing, haemoptysis. The main radiological features (in the case of pulmonary TB) being: hilar enlargement, consolidation, lymph node enlargement, lung cavitation and collapse.

In a second aspect not part of the invention there is provided a composition comprising a plurality of antibodies or antigen-binding fragments thereof that binds to each of CD4, IFN-γ and HLA-DR and wherein the plurality comprises antibodies or antigen-binding fragments thereof that are individually specific for each of CD4, IFN-γ and HLA-DR.

In other words, the composition as a whole is able to bind to each of CD4, iFN-γ and HLA-DR and comprises antibodies or antigen-binding fragments thereof that are individually able to bind only one of CD4, IFN-γ and HLA-DR.

In an additional or alternative embodiment the plurality of antibodies or antigen-binding fragments thereof additionally binds to CD3 and additionally comprises antibodies or antigen-binding fragments thereof that are individually specific for CD3.

In an additional or alternative embodiment the plurality of antibodies or antigen-binding fragments thereof additionally binds to CDS and additionally comprises antibodies or antigen-binding fragments thereof that are individually specific for CD8. In an additional or alternative embodiment the plurality of antibodies or antigen-binding fragments thereof additionally binds to a live or dead cell marker and additionally comprises antibodies or antigen-binding fragments thereof that are individually specific for a live or dead cell marker.

The antigen-binding fragment may be selected from the group consisting of Fv fragments (e.g. single chain Fv and disulphide-bonded Fv), Fab-like fragments (e.g, Fab fragments, Fab' fragments and F(ab)₂ fragments), single variable domains (e.g. V_{H} and V_{L}. domains) and domain antibodies (dAbs, including single and dual formats [*i.e.* dAb-linker-dAb]).

Also described but not included within the scope of the invention are modified versions of antibodies and an antigen-binding fragments thereof, e.g. modified by the covalent attachment of polyethylene glycol or other suitable polymer.

Methods of generating antibodies and antibody fragments are well known in the art.

For example, antibodies may be generated via any one of several methods which employ induction of in *vivo* production of antibody molecules, screening of immunoglobulin libraries (Orlandi. et al, 1989. Proc. Natl, Acad. Sci, U.S.A. 86:3833-3837; Winter et al., 1991, Nature 349:293-299) or generation of monoclonal antibody molecules by cell lines in culture. These include, but are not limited to, the hybridoma technique, the human B-cell hybridoma lechnique, and the Epstein-Barr virus (EBV)-hybridoma technique (Kohier et al., 1975. Nature 256:4950497: Kozbor et al., 1985. J. Immunol. Methods 81:31-42; Cote et al., 1983. Proc. Natl. Acad. Sci. USA 80:2026-2030; Cole et al., 1984. Mol,.Cell. Biol. 62:109-120).

Suitable monoclonal antibodies to selected antigens may be prepared by known techniques, for example those disclosed in *"*Monoclonal Antibodies: A manual of techniques", H Zola (CRC Press, 1988) and in *"*Monoclonal Hybridoma Antibodies: Techniques and Applications", J G R Hurrell (CRC Press, 1982).

Antibody fragments can be obtained using methods well known in the art (see, for example, Harlow & Lane, 1988, "Antibodies: A Laboratory Manual", Cold Spring Harbor Laboratory, New York). For example, antibody fragments according to the present invention can be prepared by proteolytic hydrolysis of the antibody or by expression in *E. coli* or mammalian cells (*e.g*. Chinese hamster ovary cell culture or other protein expression systems) of DNA encoding the fragment. Alternatively, antibody fragments can be obtained by pepsin or papain digestion of whole antibodies by conventional methods.

In certain embodiments the plurality of antibodies or antigen-binding fragments thereof is fixed to a solid support.

In certain embodiments the antibodies or antigen-binding fragments thereof with a particular specificity are separately detectable to those with a different specificity.

In other words an antibody or antigen-binding fragment thereof which is specific for a particular target can be detected as a separate entity to an antibody or antigen-binding fragment thereof which is specific for a different target. In this context 'target' refers to any of CD3, CD4, CD8, IFN-γ or HLA-DR.

In a preferred embodiment the antibodies or antigen-binding fragments thereof are visually detectable.

The antibodies or antigen-binding fragments thereof may be labelled in order to allow their detection. For example, they may be labelled with a fluorescent label (e.g. a fluorophore) or with a radio label.

Multiple examples of suitable labels such as fluorophores are well known in the art.

Fluorophores of interest include, but are not limited to fluorescein dyes (e.g. fluorescein dT, 5-carboxyfluorescein (5-FAM), 6-carboxyfluorescein (6-FAM), 2',4',1,4,-tetrachlorofluorescein (TET), 2',4',5',7',1,4-hexachlorofluorescein (HEX), and 2',7'-dimethoxy-4',5'-dichloro-6-carboxyfluorescein (JOE)), cyanine dyes such as Cy5, dansyl derivatives, rhodamine dyes (e.g. tetramethy)-6-carboxyrhodamine (TAMRA). ATTO dyes (such as ATTO 647N) and tetrapropano-6-carboxyrhodamine (ROX)), DABSYL, DABCYL cyanine, such as Cy3, anthraquinone, nitrothiazole, and nitroimidazole compounds, or other non-intercalating dyes.

Preferred possible fluorophores include, amongst others, BD Horizon^{™} violet laser dyes e.g. BD Horizon^{™}V450, Alexa Fluor ^{®} dyes e.g. Alexa Fluor ^{®} 488, Fluorescein isothiocyanate (FITC), R-phycoerythrin (PE), PECy^{™}5, PerCP, PerCPCy^{™}5.5, PE-Cy^{™}7, Allophycocyanin (APC), APC-Cy^{™}7,APC-H7, Qdot dyes e.g. Qdot 605.

Particularly preferred fluorophores include, but are not limited to PE-CF594, AF700, QDot605, Qdot655, PE-Cy7, PerCP-Cy5.5, APC-Cy7, BV570, V450, PE, FITC, APC, Biotin, PE-Cy5.

Exemplary fluorophores are used as in the following antibodies used in the methods described herein: LIVE/DEAD Fixable Deal Cell Kit, near Infa-Red (Invitrogen), CD3 Alex-Fluor 700, CD4 BV510, CD8 BV605, HLA-DR PerCP-Cy5.5, IFNγ BV421.

As used herein, "fluorophore" (also referred to as fluorochrome) refers to a molecule that, when excited with light having a selected wavelength, emits light of a different wavelength.

In a third aspect not part of the invention the composition of the second aspect is for use in determining the risk of a latent TB infection progressing to an active TB infection.

In a fourth aspect not part of the invention the composition of the second aspect is for use in treating a TB infection wherein the use comprises identifying if an individual is at risk of developing an active TB infection and subsequently administrating the most appropriate preventative treatment for that infection. For example, if the individual is shown to be at high risk of developing active TB, then treatment appropriate for treating or preventing active TB will be administered.

Examples of preventative treatment for active TB which might be administered in any aspect of the invention are isoniazid and/or rifampicin. These may be administered for 3 months, for example,

in a fifth aspect not part of the invention there is provided a method of treating a subject determined to be at risk of developing an active TB infection comprising:
(a) conducting the method of the first aspect; and
(b) administrating the most appropriate preventative treatment to the subject depending on the outcome of the step (a)

For example, if the individual is shown by step (a) to have a high risk of progressing from a latent TB infection to active TB, then an appropriate treatment will be administered in step (b) (e.g. a treatment for treating or preventing active TB will be administered in step (b)).

in a sixth aspect not part of the invention there is provided a method of stratifying an individual for preventative treatment of active TB infection comprising:
(a) conducting the method of the first aspect; and
(b) stratifying individuals determined to be at risk of developing active TB infection for the most appropriate preventative treatment depending on the outcome of the step (a)

In a seventh aspect not part of the invention there is provided a kit for determining tuberculosis (TB) infection status in an individual comprising:
(i) a composition comprising a plurality of antibodies or antigen-binding fragments thereof that binds to each of CD4, IFN-γ and HLA-DR and wherein the plurality comprises antibodies or antigen-binding fragments thereof that are individually specific for each of CD4, IFN-γ and HLA-DR;
(ii) instructions for use.

In other words, the composition (i) of the kit is a composition which as a whole is able to bind to each of CD4, IFN-γ and HLA-DR and comprises antibodies or antigen-binding fragments thereof that are individually able to bind only one of CD4, IFN-γ and HLA-DR.

In an eighth aspect not part of the invention there is provided a kit for determining tuberculosis (TB) infection status in an individual comprising:
(i) a composition comprising a plurality of antibodies or antigen-binding fragments thereof that binds to each of CD4 and HLA-DR and wherein the plurality comprises antibodies or antigen-binding fragments thereof that are individually specific for each of CD4 and HLA-DR;
(ii) a composition comprising a plurality of antibodies or antigen-binding fragments thereof that bind to IFN-γ and wherein the plurality comprises antibodies or antigen-binding fragments thereof that are individually specific for IFN-γ; and
(iii) instructions for use.

In other words, the composition (i) of the kit is a composition which as a whole is able to bind to each of CD4 and HLA-DR and comprises antibodies or antigen-binding fragments thereof that are individually able to bind only one of CD4 and HLA-DR; and the composition (ii) of the kit is a composition which as a whole is able to bind to IFN-γ and comprises antibodies or antigen-binding fragments thereof that are individually able to bind only IFN-γ.

In an additional or alternative embodiment of the kit of the seventh and eight aspects, the plurality of antibodies or antigen-binding fragments of the composition of (i) and/or (ii) is additionally able to bind to one or more of CD3, CD8, and/or a live or dead cell marker and additionally comprises antibodies or antigen-binding fragments thereof that are individually specific for the one or more of CD3, CD8, and/or a live or dead cell marker.

In an additional or alternative embodiment of the kit of the seventh and eight aspects, this kit additionally comprises one or more of: one or more TB antigens; a fixation and permeabilisation kit; a golgi inhibitor; a live and/or dead cell discriminator, a positive control antigen/s.

Example TB antigens are described above. Example dead cell discriminators (e.g. dead cell markers) are described above.

Example positive controls for use with any aspect of the invention include: PMA-Ionomycin for stimulating the sample, PMA plus calcium ionophore, lipopolysaccharide (LPS), staphylococcal enteroloxin B (SEB), Anti CD3 plus Anti CD28, phytohemagglutinin (PHA). Activated human cell seis that have been screened for cytokine production may also be used.

In a ninth aspect not part of the invention there is provided the use of a kit of the seventh or eighth aspect in a method of the first aspect.

### Definitions and abbreviations

By "CD3", "CD4" and "CD8" we refer to cluster of differentiation 3, 4 and 8, respectively.

By "IFN-γ" we refer to interferon-gamma.

By "HLA-DR" we refer to Human Leukocyte Antigen - antigen D Related.

By "+" we mean positive. In other words the particular immunological molecule/marker the + is associated with is present.

By "-" we mean negative. In other words the particular immunological molecule/marker the - is associated with is absent.

By "antibody" we include substantially intact antibody molecules, as well as chimaeric antibodies, humanised antibodies, human antibodies (wherein at least one amino acid is mutated relative to the naturally occurring human antibodies), single chain antibodies, bispecific antibodies, antibody heavy chains, antibody light chains, homodimers and heterodimers of antibody heavy and/or light chains, and antigen binding fragments and derivatives of the same.

By "antigen-binding fragment" we mean a functional fragment of an antibody that is capable of binding to a particular antigen.

The term "individual" encompasses all organisms. Examples of those organisms include mammals such as humans, cows, badgers, dogs, cats, goats, sheep, and pigs. Preferably the individual is human.

Examples embodying an aspect of the invention will now be described with reference to the following figures in which:
**Figure 1** shows example flow plots showing HLA-DR vs IFNγ expression (A) in PPD-stimulated CD4+ T cells, and (B) histograms showing distribution of HLA-DR expression in PPD-specific CD4+IFNγ + T cells (gate indicates % positive), from 2 LTBI individuals with TB contact (left panels) and 2 individuals without TB contacts (right panels).
**Figure 2** shows dot plot (Å) and ROC curve (B) showing the difference in %HLA-DR expression in PPD-specific CD4+ IFNγ+ T cells in LTBI individuals with or without TB contacts.
**Figure 3** shows measurement of the CD27 MFI (TAM-TB; A), CD45RA-CD27- (B) and CD45RA-CD27+ (C) signatures in LTBI subjects with or without TB contact.
**Figure 4** shows the results from stimulated PBMCs from 46 IGRA-positive individuals from a cohort with PPD and analysed using the signature of the invention.
**Figure 5** shows a gating strategy for identification of Mtb-specific CD4+ IFNγ+ T cells which are positive for HLA-DR.
**Figure 6** shows percentage of HLA-DR+ cells in the CD3+ CD4+ IFNγ+ cell population for IGRA+ve subjects who progressed to active TB within 2 years of IGRA testing (progressors) and IGRA+ subjects who did not progress to TB within that time-frame (non-progressors).

### Examples

### Example 1

This method works as an immune based test which is able to identify individuals with LTBI who have a greater risk of development to ATB. Thus, the test can be used to risk stratify patients with LTBI for preventative treatment.

### Eligibility for the test:

Individuals who have been diagnosed as having latent TB, i.e. already have had a positive IGRA/TST test for TB infection, and an absence of clinical/radiological features of TB.

### Samples:

A blood sample may be used (> 5 ml collected in heparin tube), from which the PBMC are extracted. Whole blood can also be used. If the assay is to be conducted at a later date, the PBMC must be cryopreserved.

### Methods

Assay steps:
1. Fresh blood/PBMC or PBMCs recovered from cryo-storage are first rested for 8 hours in a 48-well culture plate, at 37 °C. 5% CO2 for 16 hours
2. After resting, the PBMC are stimulated with three different conditions:
   - Mycobaterium tuberculosis (Mtb) antigens (PPD at 16.67 ug/ml or RD-1 antigens such as: ESAT-6, CFP-10, Rv3615c, Rv3879c each at 10 ug/ml);
   - unstimulated;
   - PMA/ionomycin (1 ug/ml and 100 ug/ml respectively)
3. After 2 hours, the release of cytokines by the immune cells is blocked by adding a Golgi-inhíbitors; a combination of Brefeldin A and Monensin at concentrations of 5 ug/ml and 2uM respectively.
4. At the end of the stimulation, the cells are recovered on ice, washed in PBS and stained with specific antibodies and viability stains
5. The cells are first stained with a viability die, then antibodies specific for the following extra-cellular markers: CD3, CD4, HLA-DR, (additional markers may also be used).
6. The cells are then fixed, permeabilised and stained with an antibody for intracellular IFNγ
7. The samples are analysed using a flow cytometry analyser as quickly as possible, then analysed using FlowJo software
8. The proportion of Mtb-specific CD4+ T cells is calculated by comparing the antigen stimulated samples to the unstimulated. Those which are cytokine positive in the antigen stimulated samples are deemed Mtb-specific, and can be analysed using the immune signature % HLA-DR+ in IFNγ+ population.
   * the percentage cut-off values for using immune signatures such as this varies between labs and cohorts. So far, the optimal cut-off for high risk vs low risk LTBI is >28% but may be higher (up to 50%) in future cohorts.

### Results

Adekambi *et al* previously demonstrated that the proportion of activated Mib-specific CD4+IFNγ+ celis can discriminate between active TB and latent TB with high accuracy, with an equivalent performance seen when either CD38, HLA-DR or Ki-67 was used as the marker of activation [1]. Since then, others have shown that the optimal differentiation between groups in HIV infected individuals is achieved by using HLA-DR as the marker of activation [2].

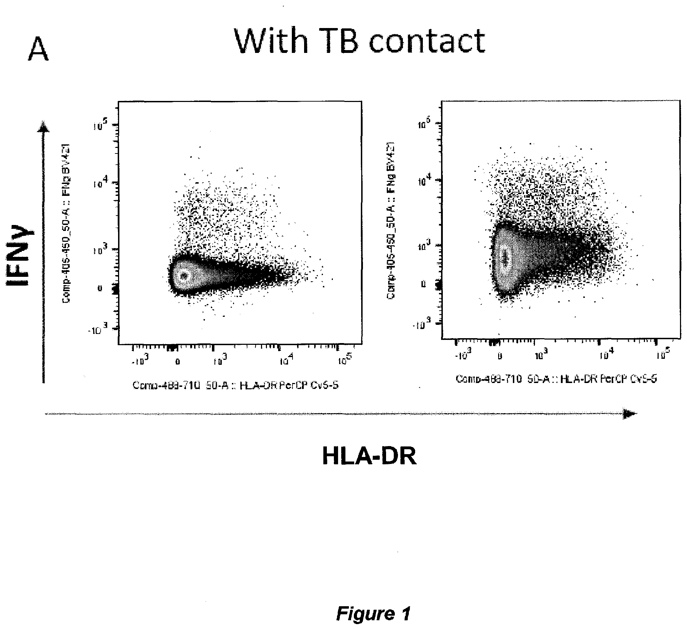

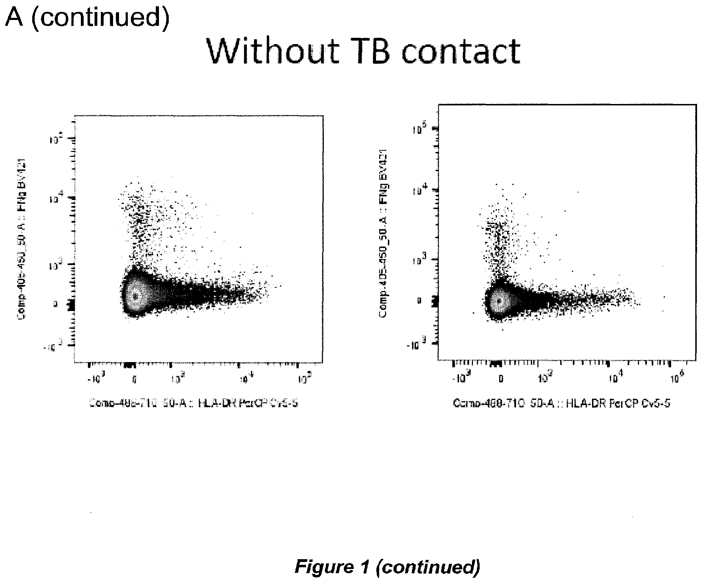

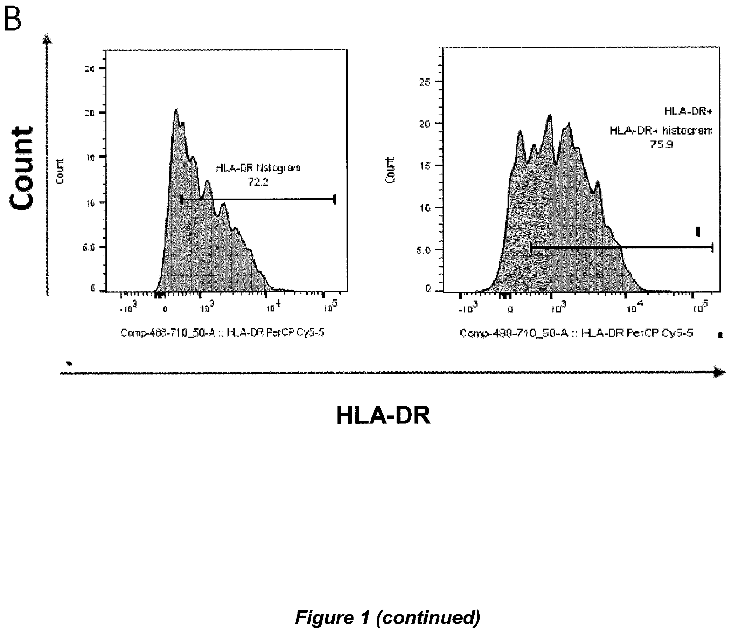

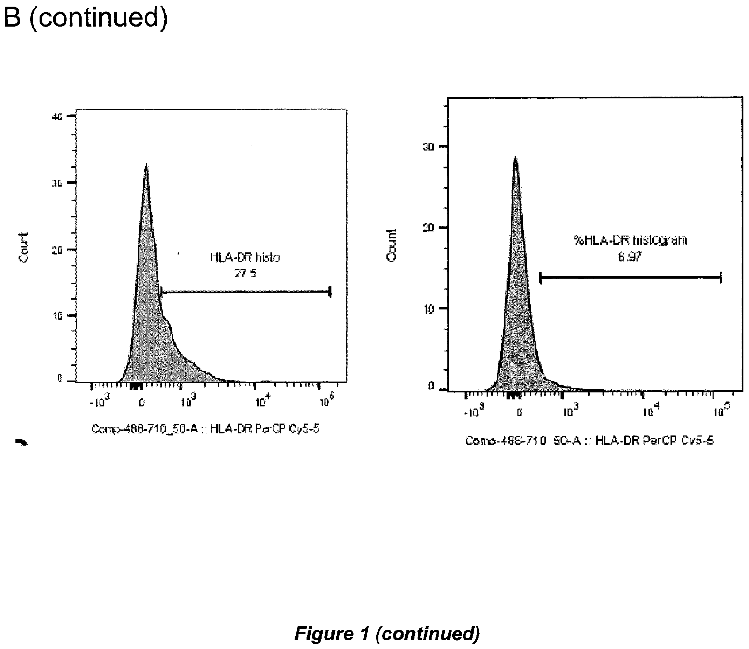

To test whether the proportion of activated Mtb-specific CD4+IFNγ+ cells is associated with a known risk factor for progression within a cohort of subjects with latent TB infection (LTBI), we evaluated the proportion of HLA-DR+ Mtb-specific T cells in Individuals with an identified recent contact with a TB case, and in those with no recent contact. We calculated the proportion of HLA-DR+ Mtb-specific CD4+IFNγ+ cells, as detailed in [1]; example dot plots from the two groups are displayed in Figure 1.

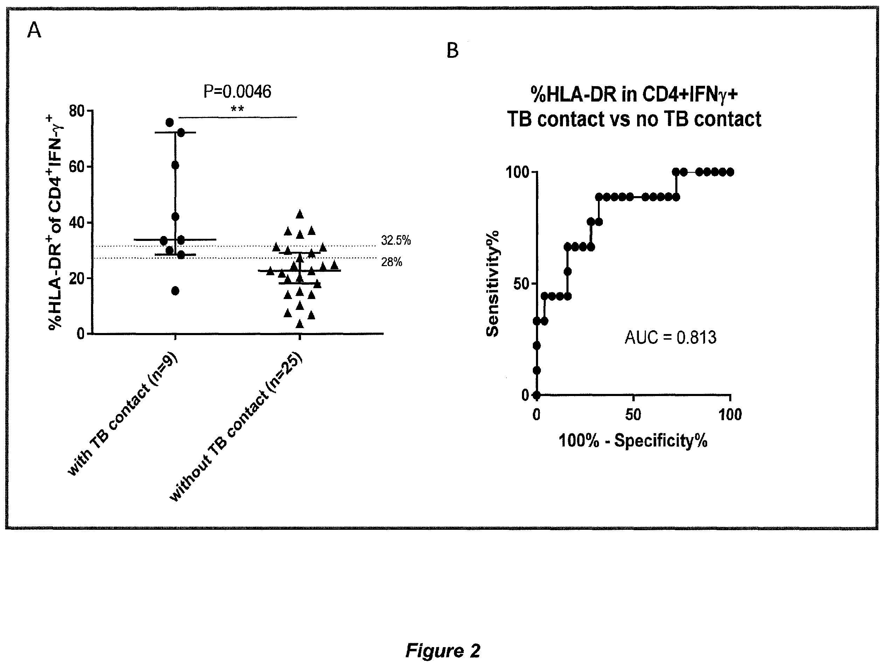

By comparing the two groups, we identified that the proportion of CD4+IFNγ+ cells which were HLA-DR+ was statistically higher in those with an identified contact with a TB case, and that this signature could discriminate between these two groups with reasonable accuracy (AUC = 0.813) (Figure 2).

This result is surprising and unexpected, because in principle one wouldn't necessarily expect a signature which distinguishes LTBI and active TB to identify risk of progression in LTBI.

In order to derive the optimal cut-off value to be used for this signature in this particular cohort, a ROC-curve was generated using the data (in this case using Prism), which provides details of the sensitivity and specificity for each data point. Using this analysis, a cut-off of 28% was found to give 89% sensitivity and 68% specificity, while a cut-off of 32.5% gave 67% sensitivity and 84% specificity for identifying those with a TB contact (Figure 2). Therefore, the cut-off could be adjusted accordingly, depending on how the user would wish to use this test in this target population.

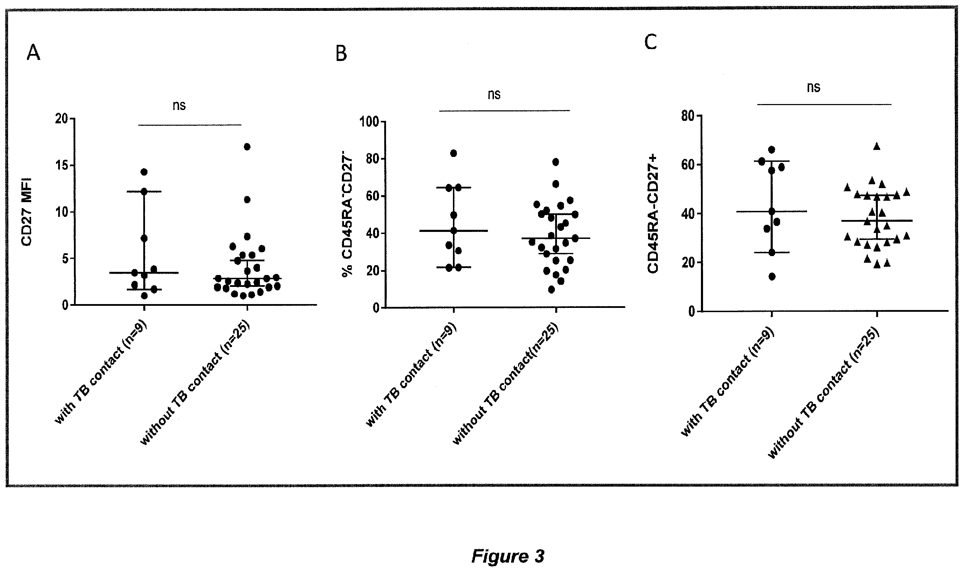

We also measured 3 other cellular immune signatures which were proposed by a German research group (the TAM-TB assay [3] and an Italian research group (the proportion of CD45RA-CD27-/+ cells within the Mtb-specific CD4+IFNγ+ population [4]) in our cohort of LTBI patients. We found that these other signatures which have also shown promise in distinguishing between active TB and LTBI were not different between the LTBI with contact and without contacts group (Figure 3), which supports our previous finding that these signatures are not different between recent and remote exposure [5], Therefore, it is not the case that all signatures which discriminate between active TB and LTBI are associated with risk of progression from LTBI to ATB, further supporting the surprising nature of the invention described herein.

### Example 2

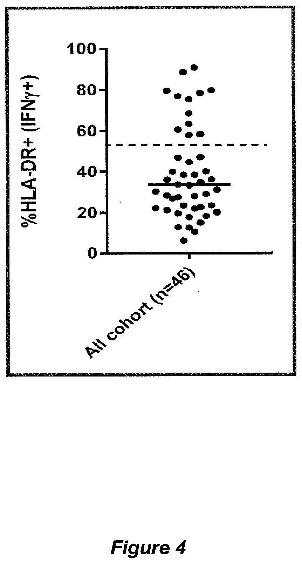

Quantification of the forward risk associated with the immune signature can be achieved by using a long-term prospective longitudinal cohort study of individuals with LTBI followed up to assess for progression to tuberculosis and correlation with baseline immune signatures. The performance of the HLA-DR signature in a cohort of individuals who were either contacts of TB cases, or recent arrivals to the UK from areas of high endemicity for TB, can be assessed. An ongoing study "PREDICT" is the largest of its kind, and over 10,000 Individuals were recruited, from which a proportion (about 90 cases, ~1% of overall cohort) developed active disease within the study period (2 years follow up). Using a nested case-control cohort approach, the progressors who were IGRA positive and for whom we have samples, were matched with 2x controls per case. So far we have stimulated PBMCs from 46 IGRA-positive individuals from this cohort with PPD and analysed them using this signature (Figure 4). This study is currently blind to the patient groups. However, we know that the cohort is split with 2:1 controls:progressors, and that the results from the signature identify two distinct populations, with 26% (of the cohort with a relatively high % of HLA-DR+, and 74% of the cohort with a relatively low % of HLA-DR+ (Figure 4), It therefore seems highly likely that those with the higher responses are those who later progress to active TB, therefore validating the use of this signature in a large cohort to produce highly specific results.

### Example 3

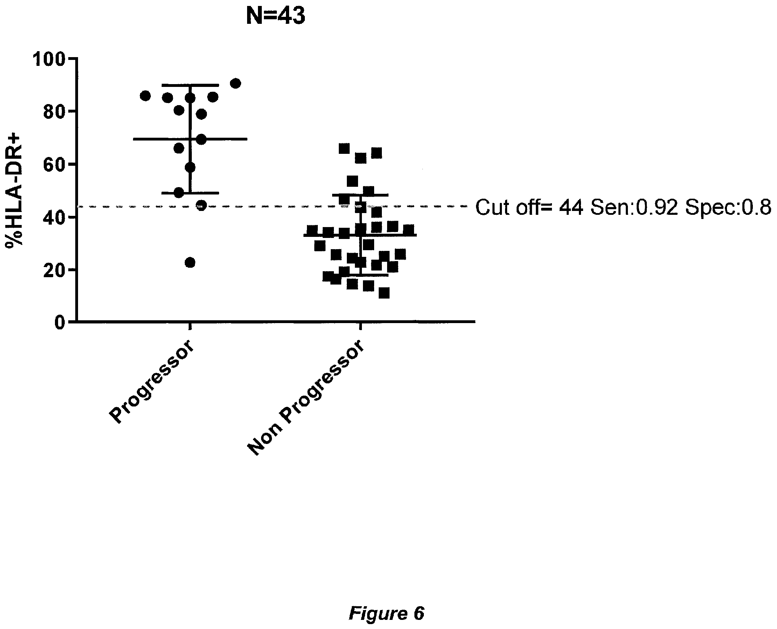

The nested case-control study described in Example 2 and the corresponding data in Figure 4 were subsequently unblinded. The results are displayed in Figure 6 and Table 1, below. These prospective clinical outcome data provide confirmatory evidence (in addition to the cross-sectional data presented in Figure 2) of the efficacy of the invention.

**Table 1:**

| | **Progressors** | **Non Progressors** |
|---|---|---|
| **Total samples tested (n=46)** | 13 | 33 |
| **Indeterminate results*** | 0 | 3 |
| **Total samples yielding evaluable results (n=43)** | 13 | 30 |
| **Total positive (cut-off > 44)** | 12 | 6 |
| **Proportion scoring positive** | 12/13 (92%) | 6/30 (20%) |
| **Diagnostic Sensitivity** | 92% | |
| **Diagnostic specificity** | | 80% |

The subjects tested in this nested case-control study were all IGRA+ and are as described in Example 2. The case-control study derives from the larger longitudinal prospective cohort study, PREDICT, also described in Example 2. The cryopreserved PBMC samples from the 46 subjects in the nested case-control study of Example 2 were thawed and stimulated with PPD under the same conditions and incubation period as described in Methods of Example 1, including addition of Golgi-inhibitors and fluorescently labelled antibodies to CD3, CD4, HLA-DR and, following fixation and permeabilisation, an additional labelled antibody for intracellular IFNγ.

Using the same gating strategy as described in Figure 5, the proportion of PPD (Mtb)-specific CD4+ IFNγ+ T cells which are positive for HLA-DR was calculated and the results are displayed in Figure 6 below. 43 of 46 samples showed IFNγ-responding cells in response to PPD stimulation; 3 samples that gave no IFNγ response to PPD and could therefore not be further analysed to identify HLA-DR+ cells within an IFNγ-positive population. Accordingly, these samples are deemed indeterminate in Table 1 and omitted from Figure 6. A cut-off of 44% HLA-DR+ cells in the CD3+ CD4+ IFNγ+ cell population was applied to the data displayed in Figure 6 to generate the summary results in Table 1.

The results in Table 1 confirm that this assay predicts which IGRA+ subjects are at risk of progression to active TB within 2 years since 12 of 13 progressors scored positive in the assay whilst only 6 of 30 non-progressors were positive. This assay can thus risk-stratify IGRA+ persons by risk of progression to active TB thereby targeting those more likely to benefit from preventative therapy.

### References

1. Adekambi, T., et al., Biomarkers on patient T cells diagnose active tuberculosis and monitor treatment response. The Journal of Clinical Investigation, 2015. 125(5): p. 1827-1838.
2. Wilkinson, K.A., et al., Activation Profile of Mycobacterium tuberculosis-Specific CD4+ T Cells Reflects Disease Activity irrespective of HIV Status. American Journal of Respiratory and Critical Care Medicine, 2016. 193(11): p. 1307-1310.
3. Portevin, D., et al., Assessment of the novel T-cell activation marker–tuberculosis assay for diagnosis of active tuberculosis in children: a prospective proof-of-concept study. The Lancet Infectious Diseases. 14(10): p. 931-938.
4. Petruccioli, E., et al., Assessment of CD27 expression as a tool for active and latent tuberculosis diagnosis. Journal of Infection, 2015. 71(5): p. 526-533.
5. Halliday, A., et al., Stratification of Latent Mycobacterium tuberculosis Infection by Cellular immune Profiling. The Journal of Infectious Diseases, 2017. 215(9): p. 1480-1487.

## Claims

1. An *in vitro* method of determining the latent tuberculosis (TB) infection status in an individual comprising:
(i) providing a sample comprising T-cells;
(ii) exposing the sample of (i) to one or more TB antigens;
(iii) identifying T-cells in the sample that are CD4 positive and secrete IFN-γ in response to TB antigens;
(iv) identifying those cells of (iii) which are also HLA-DR positive; and
(v) calculating the cells identified in (iv) as a percentage of those identified in (iii);
wherein the identification of cells in (iv) and/or the percentage of cells calculated in (v) correlates to latent TB infection status of the individual, and wherein steps (iii) and (iv) can be carried out either sequentially or simultaneously;
wherein the latent TB infection status determined by the method corresponds to the risk of the latent TB infection progressing to an active TB infection; and wherein the risk of the latent TB infection progressing to an active TB infection is proportional to the percentage value calculated in step (v) of the method.

2. The method of claim 1 wherein there is a high risk of the latent TB infection progressing to an active TB infection when the percentage calculated in step (v) is greater than a cut off value, optionally wherein the cut off value is a value between 10% and 50%, between 20% and 40%, between 25% and 40%, between 20% and 35%, between 25% and 35%, or between 25% and 30%.

3. The method of any previous claim wherein the latent TB infection status determined by the method corresponds to the amount of time elapsed since the individual was originally infected with TB, optionally wherein the time elapsed since the individual was originally infected with TB is inversely proportional to the percentage value calculated in step (v).

4. The method of any previous claim wherein the cells identified in step (iii) are additionally CD3 positive.

5. The method of any previous claim wherein the cells identified in step (iii) are additionally CD8 negative.

6. The method of any previous claim wherein the T-cells identified in step (iii) are identified as live T-cells, preferably by use of a dead cell marker.

7. The method of any previous claim wherein an additional step (ii-a) is performed between steps (ii) and (iii) to block the release of cytokines, preferably by adding a golgi-inhibitor.

8. The method of any previous claim wherein the sample is a blood sample (preferably a PBMC sample), a bronochoalveolar lavage (BAL) sample or a cerebral spinal fluid (CSF) sample.

9. The method of any previous claim wherein steps (iii) and/or (iv) are performed by multiparameter flow cytometry.

10. The method of any previous claim wherein an additional step is first performed in order to determine that the sample is obtained from an individual infected with latent TB.

11. The method of claim 10 wherein the additional step is performed using an ELISpot platform, an interferon gamma release assay (IGRA) and/or a tuberculin skin test.

12. The method of claim 10 or 11 wherein there is an absence of clinical and/or radiological features of active TB in the individual.

13. The method of any of claims 1-12 wherein the TB antigens comprise PPD and/or RD-1 antigens.

14. The method of any of claims 1-12 wherein the TB antigens comprise one or more of ESAT-6, CFP-10, Rv3615c, and Rv3879c.

## Patentansprüche

1. *In*-*vitro*-Verfahren zum Bestimmen des latenten Tuberkuloseinfektionsstatus (TB-Infektionsstatus) bei einem Individuum, umfassend:
(i) Bereitstellen einer Probe, umfassend T-Zellen;
(ii) Aussetzen der Probe von (i) einem oder mehreren TB-Antigenen;
(iii) Identifizieren von T-Zellen in der Probe, die CD4-positiv sind und IFN-γ als Reaktion auf TB-Antigene sezernieren;
(iv) Identifizieren jener Zellen von (iii), die ebenso HLA-DR-positiv sind; und
(v) Berechnen der in (iv) identifizierten Zellen als Prozentsatz der in (iii) identifizierten;
wobei die Identifizierung von Zellen in (iv) und/oder der in (v) berechnete Prozentsatz von Zellen mit dem latenten TB-Infektionsstatus des Individuums korreliert, und wobei die Schritte (iii) und (iv) entweder nacheinander oder gleichzeitig durchgeführt werden können;
wobei der durch das Verfahren bestimmte latente TB-Infektionsstatus dem Risiko entspricht, dass die latente TB-Infektion zu einer aktiven TB-Infektion fortschreitet; und wobei das Risiko, dass die latente TB-Infektion zu einer aktiven TB-Infektion fortschreitet, proportional zu dem in Schritt (v) des Verfahrens berechneten Prozentwert ist.

2. Verfahren nach Anspruch 1, wobei ein hohes Risiko besteht, dass die latente TB-Infektion zu einer aktiven TB-Infektion fortschreitet, wenn der in Schritt (v) berechnete Prozentsatz größer als ein Schwellwert ist, optional wobei der Schwellwert ein Wert zwischen 10 % und 50 %, zwischen 20 % und 40 %, zwischen 25 % und 40 %, zwischen 20 % und 35 %, zwischen 25 % und 35 % oder zwischen 25 % und 30 % ist.

3. Verfahren nach einem der vorhergehenden Ansprüche, wobei der durch das Verfahren bestimmte latente TB-Infektionsstatus der Menge an verstrichener Zeit, seit das Individuum ursprünglich mit TB infiziert wurde, entspricht, optional wobei die verstrichene Zeit, seit das Individuum ursprünglichen mit TB infiziert wurde, umgekehrt proportional zu dem in Schritt (v) berechneten Prozentwert ist.

4. Verfahren nach einem der vorhergehenden Ansprüche, wobei die in Schritt (iii) identifizierten Zellen zusätzlich CD3-positiv sind.

5. Verfahren nach einem der vorhergehenden Ansprüche, wobei die in Schritt (iii) identifizierten Zellen zusätzlich CD8-negativ sind.

6. Verfahren nach einem der vorhergehenden Ansprüche, wobei die in Schritt (iii) identifizierten T-Zellen als lebende T-Zellen identifiziert werden, bevorzugt durch Verwendung eines Markers für tote Zellen.

7. Verfahren nach einem der vorhergehenden Ansprüche, wobei ein zusätzlicher Schritt (¡¡-a) zwischen den Schritten (ii) und (iii) durchgeführt wird, um die Freisetzung von Cytokinen zu blockieren, bevorzugt durch Zugeben eines Golgi-Inhibitors.

8. Verfahren nach einem der vorhergehenden Ansprüche, wobei die Probe eine Blutprobe (bevorzugt eine PBMC-Probe), eine Probe einer Bronochoalveolarspülungsprobe (BAL-Probe) oder eine Probe eines Gehirn-Rückenmarks-Fluids (CSF-Probe) ist.

9. Verfahren nach einem der vorhergehenden Ansprüche, wobei die Schritte (iii) und/oder (iv) durch Multiparameter-Durchflusszytometrie durchgeführt werden.

10. Verfahren nach einem der vorhergehenden Ansprüche, wobei ein zusätzlicher Schritt zuerst durchgeführt wird, um zu bestimmen, dass die Probe von einem mit latenter TB infizierten Individuum erhalten ist.

11. Verfahren nach Anspruch 10, wobei der zusätzliche Schritt unter Verwendung einer ELISpot-Plattform, einer Interferon-Gamma-Freisetzungsuntersuchung (IGRA) und/oder eines Tuberkulin-Hauttests durchgeführt wird.

12. Verfahren nach Anspruch 10 oder 11, wobei eine Abwesenheit von klinischen und/oder radiologischen Merkmalen von aktiver TB bei dem Individuum besteht.

13. Verfahren nach einem der Ansprüche 1 bis 12, wobei die TB-Antigene PPD- und/oder RD-1-Antigene umfassen.

14. Verfahren nach einem der Ansprüche 1 bis 12, wobei die TB-Antigene eines oder mehrere von ESAT-6, CFP-10, Rv3615c und Rv3879c umfassen.

## Revendications

1. Procédé *in vitro* de détermination du statut d'infection tuberculeuse (TB) latente chez un individu comprenant :
(i) la fourniture d'un échantillon comprenant des cellules T ;
(ii) l'exposition de l'échantillon de (i) à un ou plusieurs antigènes TB ;
(iii) l'identification des cellules T dans l'échantillon qui sont CD4 positives et sécrètent de l'IFN-γ en réponse aux antigènes TB ;
(iv) l'identification des cellules de (iii) qui sont également positives pour HLA-DR ; et
(v) le calcul des cellules identifiées en (iv) en pourcentage de celles identifiées en (iii) ;
l'identification des cellules en (iv) et/ou le pourcentage de cellules calculé en (v) étant corrélés au statut d'infection TB latente de l'individu, et les étapes (iii) et (iv) pouvant être effectuées séquentiellement ou simultanément ;
le statut d'infection TB latente déterminé par le procédé correspondant au risque de l'évolution de l'infection TB latente vers une infection TB active ; et le risque de l'évolution de l'infection TB latente vers une infection TB active étant proportionnel à la valeur en pourcentage calculée à l'étape (v) du procédé.

2. Procédé selon la revendication 1, il existe un risque élevé d'évolution de l'infection TB latente vers une infection TB active lorsque le pourcentage calculé à l'étape (v) est supérieur à une valeur seuil, éventuellement la valeur seuil étant une valeur comprise entre 10 % et 50 %, entre 20 % et 40 %, entre 25 % et 40 %, entre 20 % et 35 %, entre 25 % et 35 % ou entre 25 % et 30 %.

3. Procédé selon l'une quelconque des revendications précédentes, le statut d'infection TB latente déterminé par le procédé correspondant à la quantité de temps écoulé depuis l'infection initiale de l'individu par la TB, éventuellement le temps écoulé depuis l'infection initiale de l'individu par la TB étant inversement proportionnel à la valeur en pourcentage calculée à l'étape (v).

4. Procédé selon l'une quelconque des revendications précédentes, les cellules identifiées à l'étape (iii) étant en outre CD3 positives.

5. Procédé selon l'une quelconque des revendications précédentes, les cellules identifiées à l'étape (iii) étant en outre CD8 négatives.

6. Procédé selon l'une quelconque des revendications précédentes, les cellules T identifiées à l'étape (iii) étant identifiées en tant que cellules T vivantes, de préférence à l'aide d'un marqueur de cellules mortes.

7. Procédé selon l'une quelconque des revendications précédentes, une étape supplémentaire (ii-a) étant réalisée entre les étapes (ii) et (iii) pour bloquer la libération de cytokines, de préférence en ajoutant un inhibiteur de Golgi.

8. Procédé selon l'une quelconque des revendications précédentes, l'échantillon étant un échantillon de sang (de préférence un échantillon de PBMC), un échantillon de lavage bronchoalvéolaire (LBA) ou un échantillon de liquide céphalo-rachidien (LCR).

9. Procédé selon l'une quelconque des revendications précédentes, les étapes (iii) et/ou (iv) étant réalisées par cytométrie de flux multiparamètre.

10. Procédé selon l'une quelconque des revendications précédentes, une étape supplémentaire étant d'abord réalisée afin de déterminer que l'échantillon est obtenu à partir d'un individu infecté par une TB latente.

11. Procédé selon la revendication 10, l'étape supplémentaire étant réalisée à l'aide d'une plateforme ELISpot, d'un test de libération d'interféron gamma (IGRA) et/ou d'un test cutané à la tuberculine.

12. Procédé selon la revendication 10 ou 11, il existe une absence de caractéristiques cliniques et/ou radiologiques de TB active chez l'individu.

13. Procédé selon l'une quelconque des revendications 1 à 12, les antigènes TB comprenant des antigènes PPD et/ou RD-1.

14. Procédé selon l'une quelconque des revendications 1 à 12, les antigènes TB comprenant un ou plusieurs parmi ESAT-6, CFP-10, Rv3615c et Rv3879c.
